# EUROPÄISCHE PATENTANMELDUNG

(11) **EP 2 457 541 A1**
(43) Veröffentlichungstag der Anmeldung: **30.05.2012**
(21) Anmeldenummer: 11009997.5
(22) Anmeldetag: 06.02.2003
(51) Int. Cl.: A61F 2/44

(54) **Zwischenwirbelimplantat**

(62) Teilanmeldung aus: 10014938.4
(71) Anmelder: Synthes GmbH, 4436 Oberdorf (CH)
(72) Erfinder: Lechmann, Beat, 2544 Bettlach (CH); Burkard, Dominique, 5014 Gretzenbach (CH); Cain, Chris M. J., Norwood S.A. 5067 (AU); Mathieu, Claude, 8001 Zürich (CH)
(74) Vertreter: Lusuardi, Werther

(57) **Zusammenfassung**

Das Zwischenwirbelimplantat umfasst einen dreidimensionalen Körper (10) mit einer Oberseite (1) und einer Unterseite (2), welche zur Anlage an die Endplatten zweier benachbarter Wirbelkörper geeignet sind, einer linken Seitenfläche (3) und einer rechten Seitenfläche (4), einer Vorderfläche (5) und einer Hinterfläche (6), einer zwischen der Oberseite (1) und Unterseite (2) liegenden, horizontalen Mittelebene (7), einer von der Vorderfläche (5) zur Hinterfläche (6) verlaufenden vertikalen Mittelebene (12) sowie einer Mehrzahl den Körper (10) durchdringenden Bohrungen (9), die zur Aufnahme von longitudinalen Fixationselementen (20) geeignet sind; wobei der dreidimensionale Körper (10) an seiner Vorderfläche (5) eine Frontplatte (8) aufweist, durch welche die Bohrungen (9) hindurchgehen und in welcher die longitudinalen Fixationselemente (20) verankerbar sind. Die Bohrungen (9) durchbohren dabei die Vorderfläche (5) des Körpers (10) nicht vollständig. Dadurch ist eine dauerhaft rigide, d.h. feste Verbindung zwischen dem Zwischenwirbelimplantat und den zu seiner Befestigung verwendeten longitudinalen Fixationselementen möglich.

## Beschreibung

Die Erfindung betrifft ein Zwischenwirbelimplantat.

Aus der GB-A-2 207 607 ist ein Zwischenwirbelimplantat bekannt, welches eine hufeisenförmige Gestalt aufweist mit einer Mehrzahl von zylindrischen Löchern. Die Löcher sind innen glatt ausgebildet und weisen lediglich einen Anschlag für die Köpfe der darin einzuführenden Knochenschrauben auf. Die Nachteile dieser Anordnung bestehen darin, dass die darin eingeführten Befestigungsschrauben nur mit ihrem Schaft im Knochen verankert werden können, ohne dass eine rigide Verbindung mit dem hufeisenförmigen Zwischenwirbelimplantat resultiert. Sobald es zu einer Schwächung der Verankerung des Schraubenschaftes im Knochen kommt, wird das Zwischenwirbelimplantat beweglich gegenüber der Schraube und es besteht die Tendenz einer Migration der Knochenschrauben unter Gefährdung der Blutgefässe. Die Lockerung des Zwischenwirbelimplantates kann zudem zu einer Pseudoarthrose führen.

Aus der US-A 2000/0010511 MICHELSON ist ein Zwischenwirbelimplantat bekannt, welches an seiner Vorderfläche zwei Bohrungen mit einem Innengewinde aufweist, in welche Knochenschrauben mit einem Gewindekopf einführbar sind. Nachteilig bei diesem Implantat ist einerseits der Umstand, dass sich die Knochenschrauben wieder lockern können und gegen ein Herausdrehen oder Herausfallen nicht gesichert sind. Anderseits besteht der weitere Nachteil, dass die Knochenschrauben vollständig am Implantatkörper selbst befestigt sind und letzterer deshalb eine relativ grosse Belastung erfährt.

Schrauben, die am anterioren oder antero-lateralen Rand des Wirbelkörpers heraustreten, riskieren Hauptgefässe wie die Aorta, Vena cava sowie Versorgungsgefässe wie lumbale Arterien und Venen zu verletzen. Die Verletzung der Hauptgefässe hat die innere Verblutung innerhalb kürzester Zeit zur Folge. Das Lockern von Schrauben ist eher möglich, wenn sie nicht winkelstabil angebracht sind.

Hier will die Erfindung Abhilfe schaffen. Der Erfindung liegt das Problem zugrunde, ein Zwischenwirbelimplantat zu schaffen, welches mit Knochenfixationsmitteln eine dauerhaft rigide Verbindung eingehen kann, so dass auch bei einer Schwächung der Knochenstruktur keine Lockerung zwischen Zwischenwirbelimplantat und Knochenfixationsmitteln auftritt. Zudem soll über eine separat ausgebildete Frontplatte eine Zuggurtung für die Knochenfixationselemente erfolgen, so dass der Implantatkörper weniger Stress, d.h. überlagerte Spannungen, erfährt.

Die Erfindung löst die gestellte Aufgabe mit einem Zwischenwirbelimplantat, umfassend einen dreidimensionalen Körper mit
A) einer Oberseite und einer Unterseite, welche zur Anlage an die Endplatten zweier benachbarter Wirbelkörper geeignet sind;
B) einer linken Seitenfläche und einer rechten Seitenfläche;
C) einer Vorderfläche und einer Hinterfläche;
D) einer zwischen der Oberseite und Unterseite liegenden, horizontalen Mittelebene;
E) einer von der Vorderfläche zur Hinterfläche verlaufenden vertikalen Mittelebene;
F) der dreidimensionale Körper an seiner Vorderfläche eine Frontplatte aufweist, durch welche eine Mehrzahl von Bohrungen hindurchgehen und in welcher longitudinale Fixationselemente verankerbar sind; und
G) die Mehrzahl von Bohrungen den Körper durchdringen und zur Aufnahme der longitudinalen Fixationselementen geeignet sind, wobei
H) die Bohrungen die Vorderfläche des Körpers nicht vollständig durchbohren.

Die durch die Erfindung erreichten Vorteile ergeben sich im wesentlichen durch die dauerhaft rigide, d.h. feste Verbindung zwischen dem Zwischenwirbelimplantat und den zu seiner Befestigung verwendeten longitudinalen Fixationselementen.

An der Vorderfläche des dreidimensionalen Körpers ist eine zur horizontalen Mittelebene des Zwischenwirbelimplantates vertikal angeordnete Frontplatte angebracht, durch welche die Bohrungen hindurchgehen und in welcher die longitudinalen Fixationselemente verankerbar sind. Dies hat gegenüber zweiteiligen Implantaten gemäss dem Stand der Technik, bei welchen in einem gesonderten Operationsschritt eine Frontplatte implantiert wird, den Vorteil, dass die Implantation des Zwischenwirbelimplantates einschrittig und damit einfacher und schneller durchführbar ist. Ein weiterer Vorteil liegt darin begründet, dass dadurch die Fixation des Zwischenwirbelimplantates möglichst frontal am Wirbelkörper erfolgt, d.h. dort wo in der Regel gutes Knochenmaterial vorhanden ist. Das Resultat ist eine anteriore Bewegungseinschränkung, ohne dass dadurch ein grösseres Risiko für die umliegenden Strukturen entsteht als dies mit einem Zwischenwirbelimplantat gemäss dem Stand der Technik der Fall ist. Die Last wird vom Zwischenwirbelimplantat unter Kompression immer noch aufgenommen und nicht durch die Frontplatte oder die Fixationsschrauben.

Bei einer besonderen Ausführungsform ist parallel zur Frontplatte eine Sicherungsplatte befestigbar, vorzugsweise mittels einer Schraubverbindung, eines Bajonettverschlusses oder eines Klickverschlusses. Die Sicherungsplatte weist zweckmässigerweise eine zentrale Bohrung auf, welche vorzugsweise mit einem Innengewinde versehen ist. Die Frontplatte weist zweckmässigerweise eine zentrale Bohrung zur Aufnahme eines Befestigungsmittels auf. Die Sicherungsplatte gestattet die simultane Sicherung sämtlicher Knochenfixationselemente.

Bei einer besonderen Ausführungsform ist mindestens eine der Bohrungen in der Frontplatte derart ausgebildet, dass ein darin aufgenommenes longitudinales Fixationselement in rigider Weise mit der Frontplatte verbindbar ist.
Zu einer rigiden Verbindung kann man beispielsweise dadurch gelangen, dass mindestens eine der Bohrungen ein Innengewinde aufweist. Eine entsprechende Knochenschraube mit einem Gewindekopf kann dann in rigider Weise mit dem Implantat verschraubt werden.
Eine Alternative dazu besteht darin, dass sich mindestens eine der Bohrungen gegen die Unterseite hin konusförmig verjüngt, so dass eine Knochenschraube mit korrespondierendem Konuskopf darin rigide verankert werden kann. Die konische Bohrung besitzt vorzugsweise einen Konuswinkel, der kleiner als der resultierende Reibungswinkel ist. Zweckmässigerweise beträgt die Konizität der konischen Bohrung 1 : 3,75 bis 1 : 20,00, vorzugsweise 1 : 5 bis 1 : 15.

Die Knochenfixationselemente können entweder einen glatten Kopf aufweisen, so dass keine rigide Verbindung mit dem Implantat erfolgt, oder aber einen Gewindekopf, Konuskopf oder Spreizkopf aufweisen, so dass eine rigide Verbindung mit dem Implantat erfolgt. In beiden Fällen werden aber die Knochenfixationselemente durch die Sicherungsplatte gegen ein späteres Herausdrehen, Herausstoßen oder Herausfallen gesichert.

Bei einer besonderen Ausführungsform ist die Frontplatte im dreidimensionalen Körper als Einsatz ausgebildet und vorzugsweise vertikal zur horizontalen Mittelebene verschieblich angeordnet, so dass sie sich gegenüber dem dreidimensionalen Körper in der Vertikalen verschieben kann. Dadurch erreicht man ein "stress shielding" (Schutz, bzw. Neutralisierung von mechanischen Spannungen), welches eine graduelle Anpassung der Endplatten an das Zwischenwirbelimplantat während des Heilungsprozesses erlaubt.
Bei einer weiteren Ausführungsform ist die Frontplatte aus einem anderen Material gefertigt als der dreidimensionale Körper, vorzugsweise aus einem metallischen Werkstoff. Als metallische Werkstoffe eigenen sich insbesondere Titan oder Titanlegierungen. Die komplette Zuggurtungsanordnung (Front platte und Schrauben) kann auch aus Implantatstahl oder hochlegierten metallische Werkstoffen wie CoCrMo der CoCrMoC gefertigt sein. Der Vorteil des Titans liegt in der Gewebeverträglichkeit und dem guten Knocheneinwachsverhalten. der Vorteil des hochlegierten metallischen Werkstoffs liegt in ihren hohen Festigkeitswerten, die filigrane Konstruktionen erlauben.

Zweckmässigerweise sind die Oberseite und/oder die Unterseite des Zwischenwirbelimplantats nicht planar, sondern vorzugsweise konvex ausgebildet. Damit kann eine bessere Anpassung an die Endplatten der benachbarten Wirbelkörper erreicht werden. Bei einer weiteren Ausführungsform sind die Seitenflächen des Zwischenwirbelimplantats im Wesentlichen alle konvex ausgebildet. Zweckmässigerweise durchbohren die Bohrungen die linke und die rechte Seitenfläche des Zwischenwirbelimplantats nicht.

Bei einer weiteren bevorzugten Ausführungsform verlaufen mindestens zwei der Bohrungen parallel. Dadurch wird die Einführbarkeit des Zwischenwirbelimplantats bei der Implantation erleichtert.

Bei einer anderen bevorzugten Ausführungsform verlaufen mindestens zwei der Bohrungen von der Vorderseite aus betrachtet divergent. Dadurch gelangt man mit den Knochenschrauben in einen Bereich des Wirbelkörpers, der im Vergleich zu dessen Zentrum eine bessere Knochenqualität aufweist.

Bei einer besonderen Ausführungsform schliessen die Achsen der Bohrungen zur horizontalen Mittelebene einen Winkel beta im Bereich von 20° bis 60°, vorzugsweise von 36° bis 48° ein. Die Achsen der Bohrungen schliessen zur vertikalen Mittelebene zweckmässigerweise einen Winkel alpha im Bereich von 10° bis 45°, vorzugsweise von 27° bis 33° ein. Damit wird ein besserer Zugang bei der Einbringung der Schrauben erreicht.

Bei einer weiteren Ausführungsform wird die horizontale Mittelebene von den Bohrungen nicht durchstossen.

Bei einer besonderen Ausführungsform ist die Oberseite und Unterseite des Körpers mit einer Strukturierung, vorzugsweise in Form von Zähnen versehen.

Das Zwischenwirbelimplantat kann als Hohlkörper ausgebildet sein, dessen Mantelflächen vorzugsweise mit Perforationen versehen sind.

Je nach den Umständen können zwei, drei, vier oder auch mehr longitudinale Fixationselemente in rigider Weise mit dem Zwischenwirbelimplantat verbunden werden, zweckmässigerweise sollte mindestens ein Fixationselement die Oberseite und mindestens ein Fixationselement die Unterseite des Zwischenwirbelimplantat durchstossen.

Vorzugsweise werden longitudinale Fixationselemente in Form von Knochenschrauben mit einem Kopf und einem Schaft verwendet, wobei der Kopf vorzugsweise mit einem Aussengewinde versehen ist, welches mit dem Innengewinde der Bohrung des Zwischenwirbelimplantats korrespondiert. Bei einer zweiten möglichen rigiden Verbindungsart kann vorzugsweise eine Knochenschraube verwendet werden, bei welcher sich der Kopf gegen den Schaft hin konisch verjüngt, wobei die Konizität des Kopfes der Konizität der Bohrung des Zwischenwirbelimplantates entspricht.

Bei einer weiteren Ausführungsform durchstossen mindestens zwei longitudinale Fixationselemente die Oberseite und mindestens zwei longitudinale Fixationselement die Unterseite. Damit erhält das Zwischenwirbelimplantat eine optimale Verankerung in den benachbarten Wirbelkörpern.

Die als Knochenschrauben ausgebildeten, longitudinalen Fixationselemente weisen vorzugsweise ein selbstbohrendes und selbstschneidendes Aussengewinde auf. Die longitudinalen Fixationselemente können auch als gewindelose Zylinderstifte ausgebildet sein, welche mit einer Bohrerspitze, vorzugsweise in Form eines Trokars versehen sind.

Eine weitere Variante besteht darin, dass die longitudinalen Fixationselemente als Spiralfedern ausgebildet sind und schliesslich können die longitudinalen Fixationselemente auch als ein- oder mehrflügelige Spiralklingen ausgebildet sein.

Das Zwischenwirbelimplantat kann aus irgendeinem körperverträglichen Material gefertigt werden, zweckmässigerweise besteht jedoch der Körper aus einem körperverträglichen Kunststoff, vorzugsweise einem unverstärkten Kunststoff. Der Vorteil gegenüber den in der Implantologie bereits bekannten faserverstärkten Kunststoffen besteht darin, dass keine Verstärkungsfasern freigelegt werden, was klinisch nachteilig ist. In einen solchen aus unverstärktem Kunststoff bestehenden Körper, können zweckmässigerweise Knochenschrauben verwendet werden, deren Aussengewinde einen Lastflankenwinkel im Bereich von 11° bis 14°, vorzugsweise von 12° bis 13° aufweist. Die vergleichsweise geringe Neigung der Lastflanke bewirkt eine hohe Klemmkraft, wodurch die Radialdehnung und die Rissgefahr im Kunststoff reduziert werden. Zweckmässigerweise weist das Aussengewinde der Knochenschrauben einen Steigungswinkel im Bereich von 6° bis 10°, vorzugsweise von 7° bis 9° auf. Dieser spezielle Steigungswinkel erzeugt eine Selbsthemmung im Gewinde und sichert damit die Knochenschraube gegen ein selbständiges Lösen.

Um die Verankerung der Knochenschraube im Kunststoffkörper zu verbessern, kann die Bohrung eine Metallhülse mit Innengewinde sein. Das Zwischenwirbelimplantat kann auch nur teilweise aus einem röntgenstrahlendurchlässigen Kunststoff und - im Bereich der Bohrungen - aus Metall, z.B. aus Titan oder Titanlegierung bestehen. Damit wird insgesamt eine bessere Führung und Verankerung der Knochenschrauben im Zwischenwirbelimplantat erreicht.

Bei einer weiteren bevorzugten Ausführungsform können die Bohrungen eine glatte Innenwand aufweisen, in welche der Gewindekopf eines metallischen, longitudinalen Fixationselementes eingeschnitten oder eingeformt werden kann.

In einer anderen Ausführungsform wird der Körper von keiner der Bohrungen vollständig durchbohrt.

In einer anderen Ausführungsform weist das Zwischenwirbelimplantat eine Sicherungsplatte auf, welche im Wesentlichen parallel zur Frontplatte am Körper oder an der Frontplatte derart befestigbar ist, dass die Bohrungen mindestens teilweise von der Sicherungsplatte abdeckbar sind.

In einer weiteren Ausführungsform ist mindestens eine der Bohrungen in der Frontplatte derart ausgebildet, dass ein darin aufgenommenes longitudinales Fixationselement in rigider Weise mit der Frontplatte verbindbar ist.

In wiederum einer weiteren Ausführungsform weist mindestens eine der Bohrungen ein Innengewinde auf.

In einer anderen Ausführungsform verjüngt sich mindestens eine Bohrung gegen die Unterseite hin konusförmig.

In einer anderen Ausführungsform ist die Frontplatte als Einsatz ausgebildet und ist vorzugsweise vertikal zur horizontalen Mittelebene angeordnet.

In wiederum einer anderen Ausführungsform ist die Frontplatte aus einem anderen Material gefertigt als der dreidimensionale Körper und ist vorzugsweise metallisch.

In einer weiteren Ausführungsform durchbohren die Bohrungen die linke Seitenfläche und die rechte Seitenfläche nicht.

In wiederum einer weiteren Ausführungsform verlaufen mindestens zwei der Bohrungen von der Vorderseite aus betrachtet divergent.

In einer anderen Ausführungsform schliessen die Achsen der Bohrungen zur horizontalen Mittelebene einen Winkel beta im Bereich von 20° bis 60°, vorzugsweise von 36° bis 48° ein.

In einer anderen Ausführungsform schliessen die Achsen der Bohrungen zur vertikalen Mittelebene einen Winkel alpha im Bereich von 10° bis 45°, vorzugsweise von 27° bis 33° ein.

In wiederum einer anderen Ausführungsform besteht das Zwischenwirbelimplantat teilweise aus einem röntgenstrahlendurchlässigen Material, vorzugsweise einem Kunststoff, und im Bereich der Bohrungen aus einem Metall, vorzugsweise auf der Basis von Titan.

In einer weiteren Ausführungsform des Zwischenwirbelimplantats ist dessen Oberseite und Unterseite mit einer Strukturierung, vorzugsweise in Form von Zähnen versehen.

In einer anderen Ausführungsform umfasst das Zwischenwirbelimplantat mindestens zwei in die Bohrungen einführbare longitudinale Fixationselemente, wobei die in die Bohrungen eingeführten longitudinalen Fixationselemente einen Kopf, eine Spitze, einen Schaft und eine Achse aufweisen, wobei die Köpfe in den Bohrungen verankerbar sind.

Die Erfindung und Weiterbildungen der Erfindung werden im Folgenden anhand der teilweise schematischen Darstellung eines Ausführungsbeispiels noch näher erläutert. Es zeigen:
Fig. 1 eine Explosionszeichnung des erfindungsgemässen Zwischenwirbelimplantates mit einer Sicherungsplatte;
Fig. 2 ein longitudinales Knochenfixationsmittel in Form einer Schraube;
Fig. 3 eine Vorderansicht des zusammengesetzten Zwischenwirbelimplantates nach Fig. 1;
Fig. 4 eine Seitenansicht des zusammengesetzten Zwischenwirbelimplantates nach Fig. 1;
Fig. 5 eine dreidimensionale Detailansicht des Körpers des Zwischenwirbelimplantates, welche die Verbindungselemente zur Frontplatte nach Fig. 6 zeigt;
Fig. 6 eine dreidimensionale Detailansicht der Frontplatte des Zwischenwirbelimplantates, welche die Verbindungselemente zum Körper nach Fig. 5 zeigt; und
Fig. 7 ein komplett montiertes Zwischenwirbelimplantat mit Frontplatte gemäss der Erfindung und mit einer Sicherungsplatte.

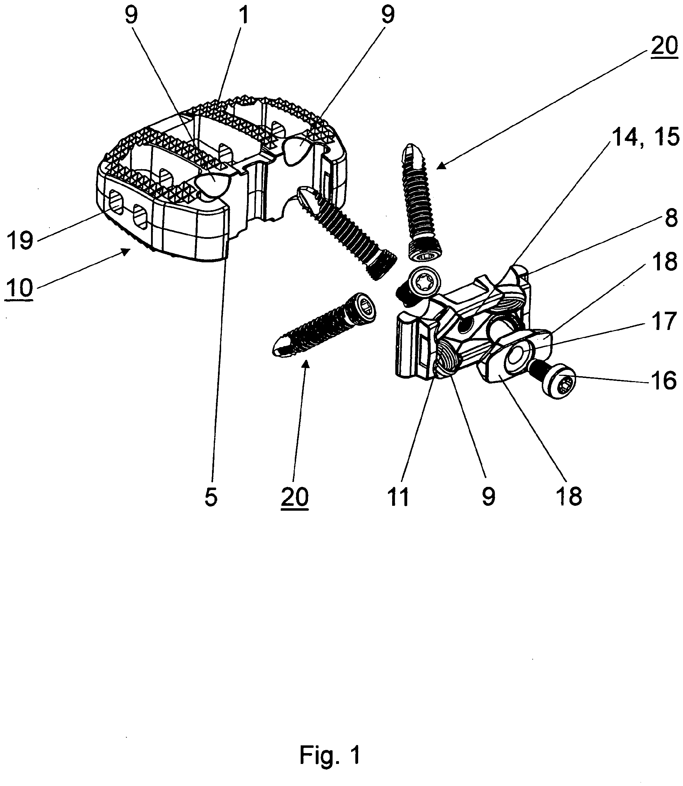

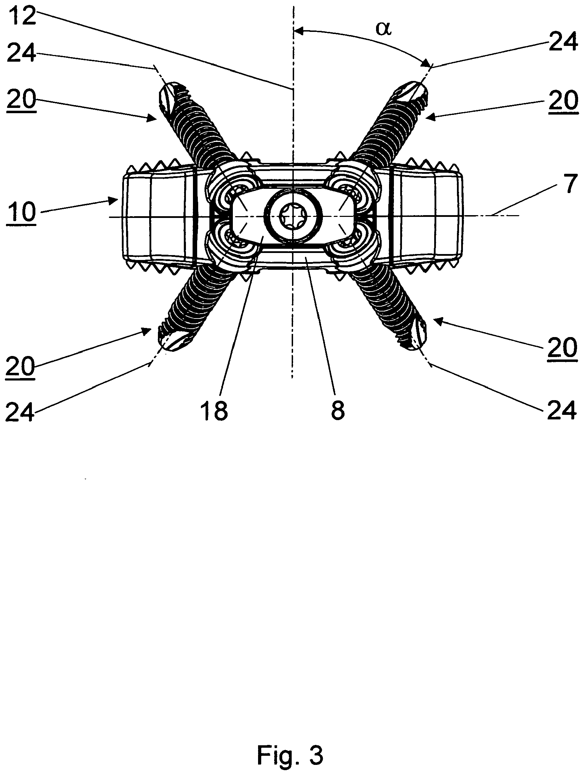

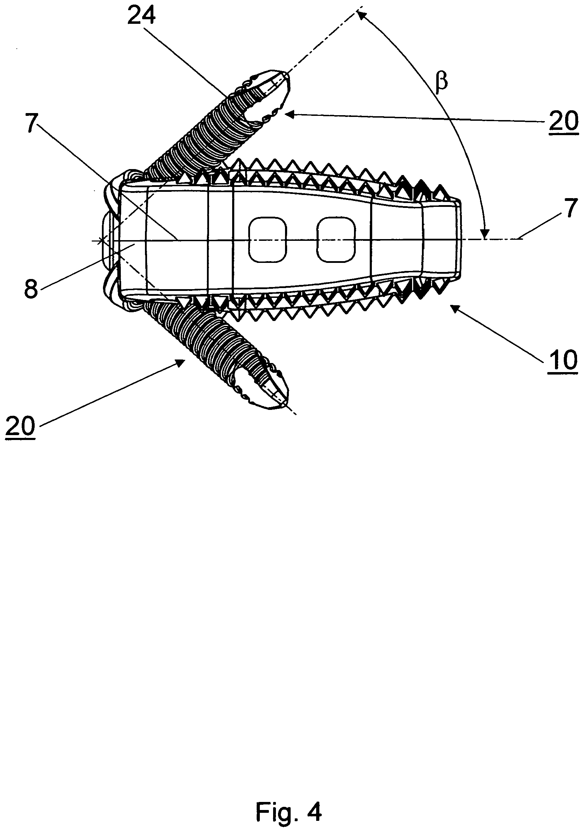

Das in den Fig. 1 - 7 dargestellte Zwischenwirbelimplantat umfasst einen dreidimensionalen Körpers 10 in Form eines Käfigs mit einer Oberseite 1 und einer Unterseite 2, welche zur Anlage an die Endplatten zweier benachbarter Wirbelkörper geeignet sind, einer linken Seitenfläche 3 und einer rechten Seitenfläche 4, einer Vorderfläche 5 und einer Hinterfläche 6, einer zwischen der Oberseite 1 und Unterseite 2 liegenden, horizontalen Mittelebene 7, einer von der Vorderfläche 5 zur Hinterfläche 6 verlaufenden vertikalen Mittelebene 12, vier den Körper 10 durchdringende Bohrungen 9, die zur Aufnahme von longitudinalen Fixationselementen 20 geeignet sind. Der dreidimensionale Körper 10 weist an seiner Vorderfläche 5 eine Frontplatte 8 auf, durch welche die Bohrungen 9 hindurchgehen und in welcher die longitudinalen Fixationselemente 20 verankerbar sind. Keine der vier Bohrungen 9 durchbohrt die Vorderfläche 5 des Körpers 10 vollständig. Der Körper 10 ist als Hohlkörper ausgebildet, dessen Mantelflächen mit Perforationen 19 versehen sind.

Das Zwischenwirbelimplantat weist im weiteren eine Sicherungsplatte 18 auf, welche mittels einer Schraubverbindung parallel zur Frontplatte 8 an der Frontplatte 8 derart befestigbar ist, dass die Bohrungen 9 teilweise von der Sicherungsplatte 18 abdeckbar sind. Die Sicherungsplatte 18 weist zu diesem Zweck eine zentrale Bohrung 17 auf. Dazu entsprechend weist die Frontplatte 8 eine zentrale Bohrung 15 mit Innengewinde 14 zur Aufnahme eines Befestigungsmittels 16 in Form einer Schraube auf. Die Sicherungsplatte 18 kann auch weggelassen werden.

Die vier Bohrungen 9 in der Frontplatte 8 weisen ein Innengewinde 11 auf, so dass die darin aufgenommenen longitudinalen Fixationselemente 20 in Form von Schrauben in rigider Weise mit der Frontplatte 8 verbindbar sind.

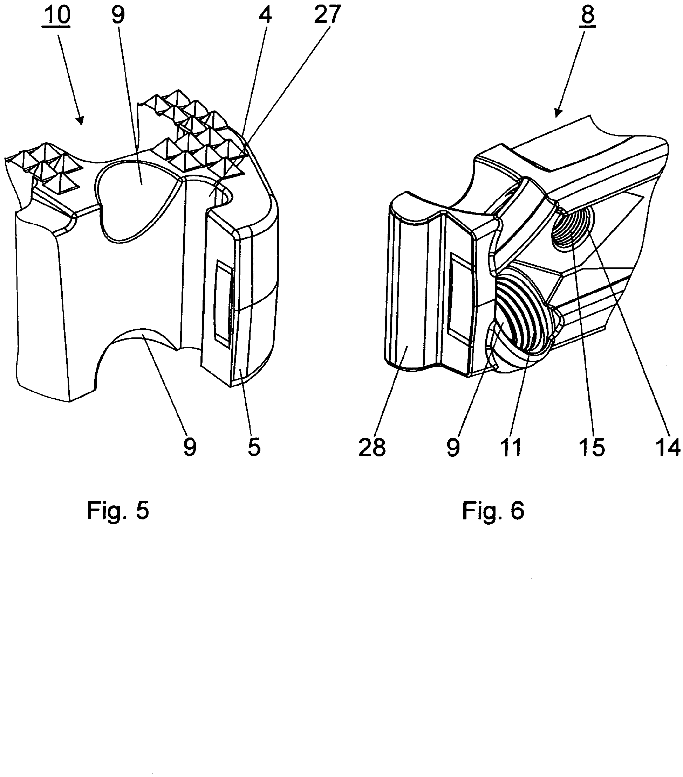

Die Frontplatte 8 besteht aus Titan und der dreidimensionale Körper 10 aus einem röntgenstrahlendurchlässigen, unverstärkten Kunststoff. Die Frontplatte 8 ist wie in den Fig. 5/6 dargestellt als Einsatz für den Körper 10 ausgebildet und vertikal zur horizontalen Mittelebene 7 verschieblich angeordnet. Der Körper 10 weist zu diesem Zweck an den Übergängen der linken Seitenfläche 3, bzw. der rechten Seitenfläche 4 (Fig. 5) zur Vorderfläche 5 ein parallel zur vertikalen Mittelebene 12 verlaufende, halbkreiszylinderförmige Nut 27 auf. Entsprechen weist die Frontplatte 8 rechts und links (Fig. 6) eine gleich verlaufende und gleich dimensionierte, halbkreiszylinderförmige Schiene 28 auf. Dadurch lässt sich die Frontplatte - bei der Herstellung des Zwischenwirbelimplantates - mit ihren beiden lateralen Schienen 28 leicht in die entsprechenden Nuten 27 des Körpers 10 einschieben und positionieren.

Die Seitenflächen 1, 2, 3, 4, 5 und 6 des Körpers 10 sind alle konvex ausgebildet.

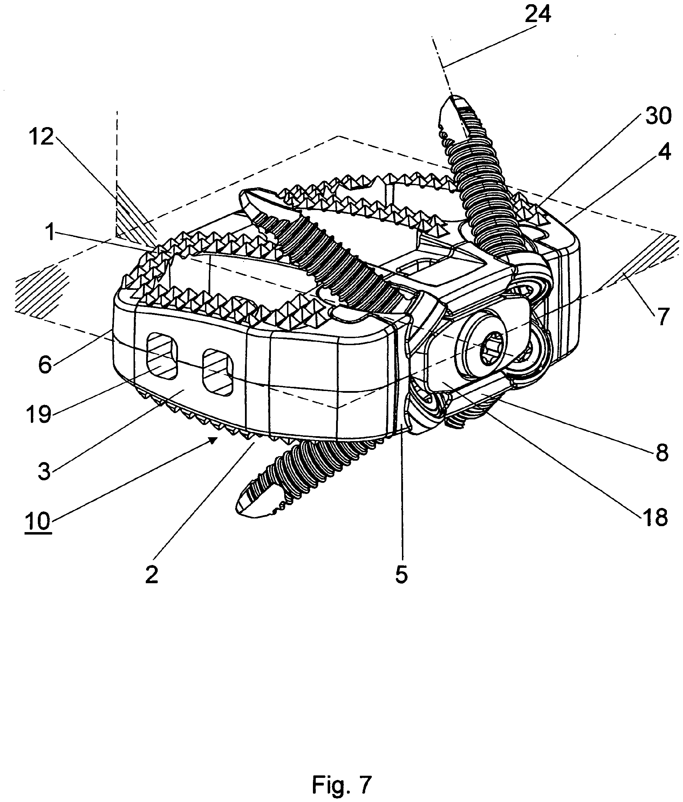

Die Bohrungen 9 durchbohren weder die linke Seitenfläche 3 noch die rechte Seitenfläche 4; auch die Vorderfläche 5 wird von den Bohrungen 9 nicht vollständig durchbohrt.
Die vier Bohrungen 9 verlaufen von der Vorderfläche 5 aus betrachtet alle divergent (Fig. 7).
Die Achsen 24 der Bohrungen 9 schliessen zur horizontalen Mittelebene 7 einen Winkel beta von 42° ein und zur vertikalen Mittelebene 12 einen Winkel alpha von 30° ein.

Die Bohrungen 9 durchstossen die horizontale Mittelebene 7 nicht, lediglich die Achsen 24 der darin eingeführten longitudinalen Fixationselementen 20 schneiden die horizontale Mittelebene 7 des Körpers 10.
Wie in Fig. 7 dargestellt ist die Oberseite 1 und Unterseite 2 des Körpers 10 mit einer Strukturierung in Form von Zähnen 30 versehen.

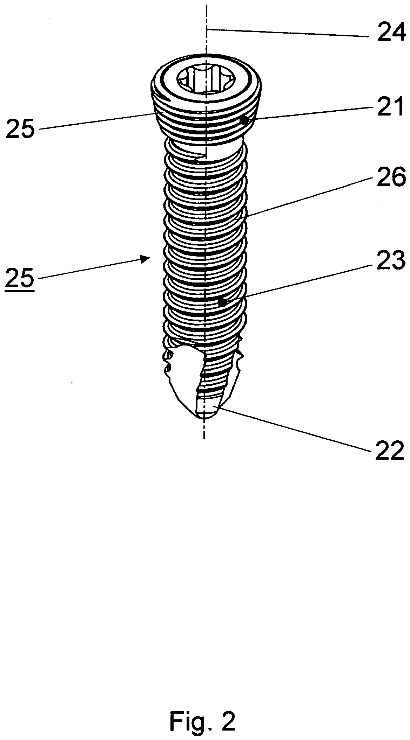

Die longitudinalen Fixationselemente 20 sind als Knochenschrauben ausgebildet. Wie in Fig. 2 dargestellt weisen die in die Bohrungen 9 eingeführten longitudinalen Fixationselemente 20 einen Kopf 21, eine Spitze 22, einen Schaft 23 und eine Achse 24 auf. Der Kopf 21 ist mit einem Aussengewinde 25 versehen, welches mit dem Innengewinde 11 der Bohrung 9 korrespondiert, so dass die Köpfe 21 in den Bohrungen 9 in rigider Weise verankerbar sind. Der Schaft 23 ist mit einem Gewinde 26 versehen ist, welches selbstbohrend und selbstschneidend ist. Der Lastflankenwinkel des Gewindes 26 beträgt 12,5° und der Steigungswinkel 8°.

Durch das Befestigen der Sicherungsplatte 18 an der Frontplatte 8 werden die Köpfe 21 der longitudinalen Fixationselemente 20 von der Sicherungsplatte 18 kontaktiert, so dass sie gegen ein Herausstossen oder Herausdrehen gesichert sind.

Wie in Fig. 7 dargestellt durchstossen zwei longitudinale Fixationselemente 20 die Oberseite 1 und zwei longitudinale Fixationselemente 20 die Unterseite 2 des Körpers 10.

Zur weiteren Erläuterung der Erfindung folgt ein kurzer Operationsbeschrieb:
a) Das Zwischenwirbelimplantat in Form eines dreidimensionalen Körpers (10) wird mittels eines geeigneten Instruments zwischen zwei benachbarte Wirbelkörper eingebracht;
b) vier longitudinale Fixationselemente 20 in Form von Knochenschrauben werden mittels eines geeigneten Zielgerätes durch die Bohrungen 9 der Frontplatte 8 in die Wirbelkörper eingeschraubt;
c) die Sicherungsplatte 18 wird mittels des Befestigungsmittels 16 in Form einer Schraube über den Köpfen 21 der longitudinalen Fixationselemente 20 an der Frontplatte befestigt, so dass die Köpfe 21 der longitudinalen Fixationselemente 20 - und damit die Schrauben selbst - zwischen der Frontplatte 8 und der Sicherungsplatte 18 gefangen und gegen eine relative Verschiebung zum Körper 10 (z.B. durch Herausfallen oder Herausdrehen) gesichert sind. Das Befestigungsmittel 16 in Form einer Schraube ist vorzugsweise mit einem Gewinde versehen, welches sich durch eine grosse Selbsthemmung auszeichnet.

## Patentansprüche

1. Zwischenwirbelimplantat, umfassend:
A) einen Körper (10), welcher eine Vorderfläche (5), eine Hinterfläche (6), eine Oberseite (1), die zur Anlage an die Endplatte eines ersten Wirbelkörper und eine Unterseite (2), die zur Anlage an die Endplatte eines zweiten Wirbelkörpers geeignet ist, wobei der Körper (10)
- eine horizontale Mittelebene (7) definiert, welche zwischen der Oberseite (1) und der Unterseite (2) liegt; und
- eine vertikale Mittelebene (12) definiert, welche zwischen der Vorderfläche (5) und der Hinterfläche (6) liegt;
B) eine Frontplatte (8), welche derart ausgebildet ist, dass sie an der Vorderfläche (5) befestigbar ist und eine Mehrzahl von Plattenbohrungen (9) aufweist, welche derart ausgebildet sind, dass sie geeignet sind einen Teil eines longitudinalen Fixationselementes (20) aufzunehmen, das in die Plattenbohrung (9) eingeführt wird, und wobei die Frontplatte (8) eine Bohrung (15) aufweist; und
C) Sicherungsmittel (18), welche derart ausgebildet sind, dass sie mit der Bohrung (15) der Fronplatte (18) kooperieren können, so dass die Sicherungsmittel (18) mindesten teilweise die Mehrzahl von Plattenbohrungen (9) abdecken und damit die Fixationselemente (20) gegen ein Herausfallen oder Herausdrehen aus den Plattenbohrungen (9) sichern.

2. Zwischenwirbelimplantat nach Anspruch 1, **dadurch gekennzeichnet, dass** dessen Oberseite (1) und/oder Unterseite (2) mit einer Strukturierung, vorzugsweise in Form von Zähnen (30), versehen ist.

3. Zwischenwirbelimplantat nach Anspruch 1, **dadurch gekennzeichnet, dass** das Sicherungsmittel (18) eine Sicherungsplatte ist.

4. Zwischenwirbelimplantat nach Anspruch 1, **dadurch gekennzeichnet, dass** es ein Fixationsmittel (20) umfasst.

5. Zwischenwirbelimplantat nach Anspruch 1, **dadurch gekennzeichnet, dass** der Körper (10) eine Körperbohrung (9) umfasst, welche im Wesentlichen mit einer der Plattenbohrung (9) der Frontplatte (8) fluchtet, wenn die Frontplatte (8) an der Vorderfläche (5) des Körpers (10) montiert ist, und dass die Plattenbohrungen (9) und die Körperbohrung (9) derart ausgebildet sind, dass sie die entsprechenden Teile eines Fixationselementes (20) aufnehmen können.

6. Zwischenwirbelimplantat nach Anspruch 1, **dadurch gekennzeichnet, dass** die Frontplatte (8) aus einem anderen Material gefertigt ist als der Körper (10).

7. Zwischenwirbelimplantat nach Anspruch 6, **dadurch gekennzeichnet, dass** der Körper (10) einen Kunststoff umfasst, vorzugsweise einem biokompatiblen Kunststoff..

8. Zwischenwirbelimplantat nach Anspruch 6, **dadurch gekennzeichnet, dass** die Frontplatte (8) ein Metall umfasst.

9. Zwischenwirbelimplantat nach Anspruch 4, **dadurch gekennzeichnet, dass** das Fixationsmittel (20) eine Knochenschraube umfasst.

10. Zwischenwirbelimplantat nach Anspruch 9, **dadurch gekennzeichnet, dass** die Knochenschraube einen Kopf (21), eine Spitze (22) und einen Schaft (23), welcher den Kopf (21) mit der Spitze (23) verbindet, umfasst.

11. Zwischenwirbelimplantat nach Anspruch 10, **dadurch gekennzeichnet, dass** der Schaft (23) mit einem Gewinde (26) versehen ist, welches vorzugsweise selbstbohrend und selbstschneidend ist.

12. Zwischenwirbelimplantat nach Anspruch 10, **dadurch gekennzeichnet, dass** ein Teil des Kopfes (21) der Knochenschraube derart ausgebildet ist, dass er in einer der Plattenbohrungen (9) aufnehmbar ist.

13. Zwischenwirbelimplantat nach Anspruch 10, **dadurch gekennzeichnet, dass** der Kopfe (21) der Knochenschraube derart ausgebildet ist, dass er zwischen dem Körper (10) und der Frontplatte (8) gefangen ist, wenn die Frontplatte (8) an der Vorderfläche (5) des Körpers (10) montiert ist.

14. Zwischenwirbelimplantat nach Anspruch 1, **dadurch gekennzeichnet, dass** die Frontplatte (8) an der Vorderfläche (5) des Körpers (10) montiert ist.

15. Zwischenwirbelimplantat nach Anspruch 1, **dadurch gekennzeichnet, dass** die Sicherungsmittel (18) eine Bohrung (17) aufweisen, welche vorzugsweise mit einem Innengewinde versehen ist.
